# NEW EUROPEAN PATENT SPECIFICATION

(11) **EP 1 013 765 B2**
(45) Date of publication and mention of the opposition decision: **13.08.2014**
(45) Mention of the grant of the patent: 05.09.2007
(21) Application number: 99125406.1
(22) Date of filing: 20.12.1999
(51) Int. Cl.: C12N 15/31, C12N 1/21, C07K 14/245, C12P 13/06, C12P 13/08

(54) **Method for producing L-amino acids**
Verfahren zur Herstellung von L-Aminosäuren
Procédé pour la préparation de L-aminoacides

(30) Priority: 23.12.1998 RU 98123511
(43) Date of publication of application: 28.06.2000
(73) Proprietor: Ajinomoto Co., Inc., Tokyo (JP)
(72) Inventor: Livshits, Vitaliy Arkadyevich, c/o GNIIGenetika, Moscow (RU); Zakataeva, Natalia Pavlovna, c/o GNIIGenetika, Moscow (RU); Aleshin, Vladimir Veniaminovich, c/o GNIIGenetika, Moscow (RU); Belareva, Alla Valentinovna, c/o GNIIGenetika, Moscow (RU); Tokhmakova, Irina Lyvovna, c/o GNIIGenetika, Moscow (RU)
(74) Representative: Strehl Schübel-Hopf & Partner

(56) References cited:
- EP-A2- 0 994 190
- WO-A-97/23597
- ZAKATAEVA ET AL.: "Characterization of a pleiotropic mutation that confers upon Escherichia coli cells resistance to high concentrations of homoserine and threonine" FASEB JOURNAL, vol. 11, no. 9, 31 July 1997 (1997-07-31), page a935 XP002135073
- DANIELS ET AL: "E.coli genomic sequence of the region from 84.5 to 86.5 minutes" EMBL DATABASE ACC NO: M87049, 1 August 1992 (1992-08-01), XP002135074
- PALMIERI ET AL.: "Threonine diffusion and threonine transport" ARCHIVES OF MICROBIOLOGY, vol. 165, no. 1, 1996, pages 48-54, XP000891665
- ZAKATAEVA ET AL.: "The novel transmembrane Escherichia coli proteins involved in the amino acid efflux" FEBS LETTERS, vol. 452, 11 June 1999 (1999-06-11), XP002135075
- ALESHIN V V ET AL: "A new family of amino-acid-efflux proteins" TIBS TRENDS IN BIOCHEMICAL SCIENCES,EN,ELSEVIER PUBLICATION, CAMBRIDGE, vol. 24, no. 4, April 1999 (1999-04), pages 133-135, XP004162806 ISSN: 0968-0004
- ZAKATAEVA ET AL.: 'Characterization of a pleiotropic mutation that confers upon Escherichia coli cells resistance to high concentrations of homoserine and threonine' FASEB JOURNAL vol. 11, no. 9, 31 July 1997, page A935, XP002135073

### SEQUENCE LISTING

<110> Ajinomoto Co., Inc.
<120>
<130> EPA-53286
<140> 99125406.1
   <141> 1999-12-20
<150> RU-98123511
   <151> 1998-12-23
<160> 4
<170> PatentIn Ver. 2.0
<210> 1
   <211> 1231
   <212> DNA
   <213> Escherichia coli
<220>
   <221> CDS
   <222> (557) .. (1171)
<400> 1
   agaaataatg tggagatcgc accgcccatc gaatgtgcca gtatatagcg tttacgccac 60
   ggaccgggct gaacctcctg ctgccagaat gccgccagat catcaacata atcattaaag 120
   cgattaacat gcccgagatg cggatcggct aacaggcgac cggaacgtcc ctgcccgcga 180
   tggtcgatga ttaagacatc aaaccccaaa tggaacaggt cataggccag ttccgcatat 240
   tttacgtagc tctcaatacg ccccgggcag atgactacca cccggtcatg gtgctgtgcg 300
   cgaaaacgga caaagcgcac cggaatgtca tccacaccag taaactctgc ttcatcacgc 360
   tgacgccaga aatcagtcag cggtcccatg gtaaaagcag caaacgcgtt ttctcttgtt 420
   tcccagtctt tttgctgctg aaacatcggg taatctgcct cttaaaccac gtaaaatcgt 480
   tttttttagc gtgcctgaca caacgctgcg acagtagcgt attgtggcac aaaaatagac 540
   acaccgggag ttcatc atg acc tta gaa tgg tgg ttt gcc tac ctg ctg aca 592
                     Met Thr Leu Glu Trp Trp Phe Ala Tyr Leu Leu Thr
                      1               5                  10
   tcg atc att tta acg ctg tcg cca ggc tct ggt gca atc aac act atg   640
   Ser Ile Ile Leu Thr Leu Ser Pro Gly Ser Gly Ala Ile Asn Thr Met
           15                  20                  25
   acc acc tcg ctc aac cac ggt tat ccg gcc ggt ggc gtc tat tgc tgg   688
   Thr Thr Ser Leu Asn His Gly Tyr Pro Ala Gly Gly Val Tyr Cys Trp
       30                  35                  40
   gct tca gac cgg act ggc gat tca tat tgt gct ggt tgg cgt ggg gtt   736
   Ala Ser Asp Arg Thr Gly Asp Ser Tyr Cys Ala Gly Trp Arg Gly Val
   45                  50                  55                  60
   ggg acg cta ttt tcc cgc tca gtg att gcg ttt gaa gtg ttg aag tgg   784
   Gly Thr Leu Phe Ser Arg Ser Val Ile Ala Phe Glu Val Leu Lys Trp
                   65                  70                  75
   gca ggc gcg gct tac ttg att tgg ctg gga atc cag cag tgg cgc gcc   832
   Ala Gly Ala Ala Tyr Leu Ile Trp Leu Gly Ile Gln Gln Trp Arg Ala
               80                  85                  90
   gct ggt gca att gac ctt aaa tcg ctg gcc tct act caa tcg cgt cga   880
   Ala Gly Ala Ile Asp Leu Lys Ser Leu Ala Ser Thr Gln Ser Arg Arg
           95                  100                 105
   cat ttg ttc cag cgc gca gtt ttt gtg aat ctc acc aat ccc aaa agt   928
   His Leu Phe Gln Arg Ala Val Phe Val Asn Leu Thr Asn Pro Lys Ser
       110                 115                 120
   att gtg ttt ctg gcg gcg cta ttt ccg caa ttc atc atg ccg caa cag   976
   Ile Val Phe Leu Ala Ala Leu Phe Pro Gln Phe Ile Met Pro Gln Gln
   125                 130                 135                 140
   ccg caa ctg atg cag tat atc gtg ctc ggc gtc acc act att gtg gtc   1024
   Pro Gln Leu Met Gln Tyr Ile Val Leu Gly Val Thr Thr Ile Val Val
                   145                 150                 155
   gat att att gtg atg atc ggt tac gcc acc ctt gct caa cgg att gct   1072
   Asp Ile Ile Val Met Ile Gly Tyr Ala Thr Leu Ala Gln Arg Ile Ala
               160                 165                 170
   cta tgg att aaa gga cca aag cag atg aag gcg ctg aat aag att ttc   1120
   Leu Trp Ile Lys Gly Pro Lys Gln Met Lys Ala Leu Asn Lys Ile Phe
           175                 180                 185
   ggc tcg ttg ttt atg ctg gtg gga gcg ctg tta gca tcg gcg agg cat   1168
   Gly Ser Leu Phe Met Leu Val Gly Ala Leu Leu Ala Ser Ala Arg His
       190                 195                 200
   gcg tgaaaaataa tgtcggatgc ggcgtaaacg ccttatccga cttactctga       1221
   Ala
   205
   agacgcgtct                                                        1231
<210> 2
   <211> 205
   <212> PRT
   <213> Escherichia coli
<400> 2
   Met Thr Leu Glu Trp Trp Phe Ala Tyr Leu Leu Thr Ser Ile Ile Leu
   1               5                   10                  15
   Thr Leu Ser Pro Gly Ser Gly Ala Ile Asn Thr Met Thr Thr Ser Leu
               20                  25                  30
   Asn His Gly Tyr Pro Ala Gly Gly Val Tyr Cys Trp Ala Ser Asp Arg
           35                  40                  45
   Thr Gly Asp Ser Tyr Cys Ala Gly Trp Arg Gly Val Gly Thr Leu Phe
       50                  55                  60
   Ser Arg Ser Val Ile Ala Phe Glu Val Leu Lys Trp Ala Gly Ala Ala
   65                  70                  75                  80
   Tyr Leu Ile Trp Leu Gly Ile Gln Gln Trp Arg Ala Ala Gly Ala Ile
                   85                  90                  95
   Asp Leu Lys Ser Leu Ala Ser Thr Gln Ser Arg Arg His Leu Phe Gln
               100                 105                 110
   Arg Ala Val Phe Val Asn Leu Thr Asn Pro Lys Ser Ile Val Phe Leu
           115                 120                 125
   Ala Ala Leu Phe Pro Gln Phe Ile Met Pro Gln Gln Pro Gln Leu Met
       130                 135                 140
   Gln Tyr Ile Val Leu Gly Val Thr Thr Ile Val Val Asp Ile Ile Val
   145                 150                 155                 160
   Met Ile Gly Tyr Ala Thr Leu Ala Gln Arg Ile Ala Leu Trp Ile Lys
                   165                 170                 175
   Gly Pro Lys Gln Met Lys Ala Leu Asn Lys Ile Phe Gly Ser Leu Phe
               180                 185                 190
   Met Leu Val Gly Ala Leu Leu Ala Ser Ala Arg His Ala
           195                 200                 205
<210> 3
   <211> 840
   <212> DNA
   <213> Escherichia coli
<220>
   <221> CDS
   <222> (187)..(804)
<400> 3
   atgccgatca ccgccagcga aatgctcagc gttaacggcg ttgggatgcg caagctggaa 60
   cgctttggca aaccgtttat ggcgctgatt cgtgcgcatg ttgatggcga tgacgaagag 120
   tagtcagcag cataaaaaag tgccagtatg aagactccgt aaacgtttcc cccgcgagtc 180
   aaatgt atg ttg atg tta ttt ctc acc gtc gcc atg gtg cac att gtg   228
          Met Leu Met Leu Phe Leu Thr Val Ala Met Val His Ile Val
          1               5                   10
   gcg ctt atg agc ccc ggt ccc gat ttc ttt ttt gtc tct cag acc gct   276
   Ala Leu Met Ser Pro Gly Pro Asp Phe Phe Phe Val Ser Gln Thr Ala
   15                  20                  25                  30
   gtc agt cgt tcc cgt aaa gaa gcg atg atg ggc gtg ctg ggc att acc   324
   Val Ser Arg Ser Arg Lys Glu Ala Met Met Gly Val Leu Gly Ile Thr
                   35                  40                  45
   tgc ggc gta atg gtt tgg gct ggg att gcg ctg ctt ggc ctg cat ttg   372
   Cys Gly Val Met Val Trp Ala Gly Ile Ala Leu Leu Gly Leu His Leu
               50                  55                  60
   att atc gaa aaa atg gcc tgg ctg cat acg ctg att atg gtg ggc ggt   420
   Ile Ile Glu Lys Met Ala Trp Leu His Thr Leu Ile Met Val Gly Gly
           65                  70                  75
   ggc ctg tat ctc tgc tgg atg ggt tac cag atg cta cgt ggt gca ctg   468
   Gly Leu Tyr Leu Cys Trp Met Gly Tyr Gln Met Leu Arg Gly Ala Leu
       80                  85                  90
   aaa aaa gag gcg gtt tct gca cct gcg cca cag gtc gag ctg gcg aaa   516
   Lys Lys Glu Ala Val Ser Ala Pro Ala Pro Gln Val Glu Leu Ala Lys
   95                  100                 105                 110
   agt ggg cgc agt ttc ctg aaa ggt tta ctg acc aat ctc gct aat ccg   564
   Ser Gly Arg Ser Phe Leu Lys Gly Leu Leu Thr Asn Leu Ala Asn Pro
                   115                 120                 125
   aaa gcg att atc tac ttt ggc tcg gtg ttc tca ttg ttt gtc ggt gat   612
   Lys Ala Ile Ile Tyr Phe Gly Ser Val Phe Ser Leu Phe Val Gly Asp
               130                 135                 140
   aac gtt ggc act acc gcg cgc tgg ggc att ttt gcg ctg atc att gtc   660
   Asn Val Gly Thr Thr Ala Arg Trp Gly Ile Phe Ala Leu Ile Ile Val
           145                 150                 155
   gaa acg ctg gcg tgg ttt acc gtc gtt gcc agc ctg ttt gcc ctg ccg   708
   Glu Thr Leu Ala Trp Phe Thr Val Val Ala Ser Leu Phe Ala Leu Pro
       160                 165                 170
   caa atg cgc cgt ggt tat caa cgt ctg gcg aag tgg att gat ggt ttt   756
   Gln Met Arg Arg Gly Tyr Gln Arg Leu Ala Lys Trp Ile Asp Gly Phe
   175                 180                 185                 190
   gcc ggg gcg tta ttt gcc gga ttt ggc att cat ttg att att tcg cgg   804
   Ala Gly Ala Leu Phe Ala Gly Phe Gly Ile His Leu Ile Ile Ser Arg
                   195                 200                 205
   tgatgccaga cgcgtcttca gagtaagtcg gataag                          840
<210> 4
   <211> 206
   <212> PRT
   <213> Escherichia coli
<400> 4
   Met Leu Met Leu Phe Leu Thr Val Ala Met Val His Ile Val Ala Leu
   1 5 10 15
   Met Ser Pro Gly Pro Asp Phe Phe Phe Val Ser Gln Thr Ala Val Ser
   20 25 30
   Arg Ser Arg Lys Glu Ala Met Met Gly Val Leu Gly Ile Thr Cys Gly
   35 40 45
   Val Met Val Trp Ala Gly Ile Ala Leu Leu Gly Leu His Leu Ile Ile
   50 55 60
   Glu Lys Met Ala Trp Leu His Thr Leu Ile Met Val Gly Gly Gly Leu
   65 70 75 80
   Tyr Leu Cys Trp Met Gly Tyr Gln Met Leu Arg Gly Ala Leu Lys Lys
   85 90 95
   Glu Ala Val Ser Ala Pro Ala Pro Gln Val Glu Leu Ala Lys Ser Gly
   100 105 110
   Arg Ser Phe Leu Lys Gly Leu Leu Thr Asn Leu Ala Asn Pro Lys Ala
   115 120 125
   Ile Ile Tyr Phe Gly Ser Val Phe Ser Leu Phe Val Gly Asp Asn Val
   130 135 140
   Gly Thr Thr Ala Arg Trp Gly Ile Phe Ala Leu Ile Ile Val Glu Thr
   145 150 155 160
   Leu Ala Trp Phe Thr Val Val Ala Ser Leu Phe Ala Leu Pro Gln Met
   165 170 175
   Arg Arg Gly Tyr Gln Arg Leu Ala Lys Trp Ile Asp Gly Phe Ala Gly
   180 185 190
   Ala Leu Phe Ala Gly Phe Gly Ile His Leu Ile Ile Ser Arg
   195 200 205

## Description

### Technical field

The present invention relates to biotechnology, and more specifically to a method for producing amino acid, especially for a method for producing L-homoserine, L-threonine, L-valine or L-leucine using a bacterium belonging to the genus *Escherichia*.

### Background Art.

The present inventors obtained, with respect to *E. coli* K-12, a mutant having mutation, *thrR* that is concerned in resistance to high concentrations of threonine or homoserine in a minimal medium (Astaurova, O.B. et al., Appl. Bioch. And Microbiol., 21, 611-616 (1985)). The mutation improved the production of L-threonine (SU Patent No. 974817), homoserine and glutamate (Astaurova, O.B. et al., Appl. Bioch. And Microbiol., 27, 556-561, 1991) by the respective *E. coli* producing strains.

Furthermore, the present inventors has revealed that the *thrR* mutation exists at 18 min on *E.* coli chromosome and that the mutation arose in ORF1 between *pexB* and ompX genes. The unit expressing a protein encoded by the ORF has been designated as *rhtA* (rht: resistance to homoserine and threonine) gene. The *rhtA* gene includes a 5'-noncoding region including SD sequence, ORF1 and a terminator. Also, the present inventors have found that a wild type rhtA gene participates in resistance to threonine and homoserine if cloned in a multicopy state and that the rhtA23 mutation is an A-for-G substitution at position -1 with respect to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457).

It has been found previously that at least two different genes which impart threonine and homoserine resistance in a multicopy state exist in *E. coli* during cloning of the *rhtA* gene. One of the genes is the *rhtA* gene, and the other gene was found to be *rhtB* gene which confers homoserine resistance (Russian Patent Application No. 98118425).

### Disclosure of the Invention

An object of the present invention is to provide a method for producing an amino acid, especially, L-homoserine, L-threonine and branched chain amino acids with a higher yield.

The inventors have found that a region at 86 min on *E. coli* chromosome, when cloned by a multicopy vector, impart resistance to L-homoserine to cells of *E. coli.* the inventors further found that there exists in the upstream region another gene, rhtC, which involves resistance to threonine, and that when these genes are amplified, the amino acid productivity of *E. coli* can be improved like the *rhtA* gene. On the basis of these findings, the present invention have completed.

Thus, the following is disclosed:
(1) A bacterium belonging to the genus *Escherichia*, wherein L-threonine resistance of the bacterium is enhanced by enhancing an activity of protein as defined in the following (A) or (B) in a cell of the bacterium:
   (A) a protein which comprises the amino acid sequence of SEQ ID NO: 4; or
   (B) a protein which comprises the amino acid sequence including deletion, substitution, insertion or addition of one or several amino acids in the amino acid sequence of SEQ ID NO: 4, and which has an activity of making a bacterium having the protein L-threonine-resistant;
(2) The bacterium according to (1), wherein L-homoserine resistance of the bacterium is enhanced by enhancing an activity of protein as defined in the following (C) or (D) in a cell of the bacterium:
   (C) a protein which comprises the amino acid sequence of SEQ ID NO: 2; or
   (D) a protein which comprises the amino acid sequence including deletion, substitution, insertion or addition of one or several amino acids in the amino acid sequence of SEQ ID NO: 2, and which has an activity of making a bacterium having the protein L-homoserine-resistant;
(3) The bacterium according to (1) or (2), wherein the activity of protein as defined in (A) or (B) is enhanced by transformation of the bacterium with DNA coding for the protein as defined in (A) or (B);
(4) The bacterium according to (2), wherein the activity of protein as defined in (C) or (D) is enhanced by transformation of the bacterium with DNA coding for the protein as defined in (C) or (D);
(5) A method for producing an amino acid, comprising the steps of:
   cultivating the bacterium as defined in any one of (1) to (4), which has an ability to produce an amino acid, in a culture medium, to produce and accumulate the amino acid in the medium, and recovering the amino acid from the medium;
(6) The method according to (5), wherein the amino acid is selected from the group consisting of L-homoserine, L-threonine and branched chain amino acids;
(7) The method according to (6), the branched chain amino acid is L-valine or L-leucine.
(8) A DNA which encode a protein defined in the following (A) or (B):
   (A) a protein which has the amino acid sequence of SEQ ID NO: 4;
   (B) a protein which has the amino acid sequence of SEQ ID NO: 4 including substitution, deletion, insertion, addition, or inversion of one or several amino acids, and has an activity of making a bacterium having the protein L-threonine-resistant.
9. The DNA of (8) which is a DNA defined in the following (a) or (b):
   (a) a DNA which comprises the nucleotide sequence of nucleotide numbers 187 to 804 in SEQ ID NO: 3;
   (b) a DNA which is hybridizable with a nucleotide sequence of nucleotide numbers 187 to 804 in SEQ ID NO: 3 or a probe prepared from the nucleotide sequence under a stringent condition, and encodes a protein having an activity of making a bacterium having the protein L-threonine-resistant; and
10. The DNA of (9) wherein the stringent condition is a condition in which washing is performed at 60 °C, and at a salt concentration corresponding to 1 x SSC and 0.1 % SDS.

The DNA fragment coding for the protein as defined in the above (A) or (B) may be referred to as "*rhtC* gene", a protein coded by the *rhtC* gene may be referred to as "*RhtC* protein", the DNA coding for the protein as defined in the above (C) or (D) may be referred to as "*rhtB* gene", a protein coded by the *rhtB* gene may be referred to as "*RhtB* protein". An activity of the RhtC protein which participate in resistance to L-threonine of a bacterium (i.e. an activity of marking a bacterium having the RhtC protein L-threonine-resistant) may be referred to as "Rt activity", and an activity of the RhtB protein which participates in resistance to L-homoserine of a bacterium (i.e. an activity of marking a bacterium having the RhtB protein L-homoserine-resistant) may be referred to as "Rh activity". A structural gene encoding the RhtC protein or RhtB protein in the *rhtC* gene or *rhtB* gene may be referred to as "*rhtC* structural gene" or "*rhtB* structural gene". The term "enhancing the Rt activity or the Rh activity" means imparting resistance to threonine or homoserine to a bacterium or enhance the resistance by means of increasing the number of molecules of the RhtC protein or RhtB protein increasing a specific activity of these proteins, or desensitizing negative regulation against the expression or the activity of these proteins or the like. The terms "DNA coding for a protein" mean a DNA of which one of strands codes for the protein when the DNA is double-stranded. The L-threonine resistance means a property that a bacterium grows on a minimal medium containing L-threonine at a concentration at which a wild-type strain thereof not grow, usually at >30 mg/ml. The L-homoserine resistance means a property that a bacterium grows on a minimal medium containing L-homoserine at a concentration at which a wild-type strain thereof not grow, usually at >5 mg/ml. The ability to produce an amino acid means a property that a bacterium produce and accumulates the amino acid in a medium in a larger amount than a wild type strain thereof.

The present invention will be explained in detail below.

### <1> DNA used for encording a protein used in the method of the invention

The first DNA fragment used for the present invention (*rhtC* gene) coding for a protein having the Rt activity and having the amino acid sequence of SEQ ID NO: 4. Specifically, the DNA may be exemplified by a DNA comprising a nucleotide sequence of the nucleotide numbers 187 to 804 of a nucleotide sequence of SEQ ID NO: 3.

The second DNA fragment used for the present invention (*rhtB* gene) coding for a protein having the Rh activity an having the amino acid sequence of SEQ ID NO: 2. Specifically, the DNA may be exemplified by a DNA comprising a nucleotide sequence of the nucleotide numbers 557 to 1171 of a nucleotide sequence of SEQ ID NO: 1.

The rhtB gene having the nucleotide sequence of SEQ ID NO: 1 corresponds to a part of sequence complement to the sequence of GenBank accession number M87049, and includes f138 (nucleotide numbers 61959-61543 of M87049) which is a known but function-unknown ORF (open reading frame) present at 86 min on *E. coli* chromosome, and 5'-and 3'- flanking regions thereof. The f138, which had only 160 nucleotides in the 5'-flanking region, could not impart the resistance to homoserine. No termination codon is present between the 62160 and 61959 nucleotides of M87049 (upstream the ORF f138). Moreover, one of the ATG codons of this sequence is preceded by a ribosomebinding site (62171-62166 in M87049). Hence, the coding region is 201 bp longer. The larger ORF (nucleotide numbers 62160 to 61546 of M87049) is designated as *rhtB* gene.

The *rhtB* gene may be obtained, for example, by infecting Mucts lysogenic strain of *E. coli* using a lysate of a lysogenic strain of *E. coli* such as K12 or W3110 according to the method in which mini-Mu d5005 phagemid is used (Groisman, E.A., et al., J. Bacteriol., 168, 357-364 (1986)), and isolating phagemid DNAs from colonies growing on a minimal medium containing kanamycin (40 µg/ml) and L-homoserine (10 mg/ml). As illustrated in the Example described below, the *rhtB* gene was mapped at 86 min on the chromosome of *E. coli*. Therefore, the DNA fragment including the *rhtB* gene may be obtained from the chromosome of *E. coli* by colony hybridization or PCR (polymerase chain reaction, refer to White, T.J. et al, Trends Genet. 5, 185 (1989)) using oligonucleotide(s) which has a sequence corresponding to the region near the portion of 86 min on the chromosome *E. coli.*

Alternatively, the oligonucleotide may be designed according to the nucleotide sequence of SEQ ID NO: 1. By using oligonucleotides having nucleotide sequences corresponding to an upstream region from the nucleotide number 557 and a downstream region from the nucleotide number 1171 in SEQ ID NO: 1 as the primers for PCR, the entire coding region can be amplified.

Synthesis of the oligonucleotides can be performed by an ordinary method such as a phosphoamidite method (see Tetrahedron Letters, 22, 1859 (1981)) by using a commercially available DNA synthesizer (for example, DNA Synthesizer Model 380B produced by Applied Biosystems). Further, the PCR can be performed by using a commercially available PCR apparatus (for example, DNA Thermal Cycler Model PJ2000 produced by Takara Shuzo Co., Ltd.) using *Taq* DNA polymerase (supplied by Takara Shuzo Co., Ltd.) in accordance with a method designate by the supplier.

The *rhtC* gene was obtained in the DNA fragment including *rhtB* gene by chance when *rhtB* was cloned as described later in the embodiments. The *rhtC* gene corresponds to a corrected, as described below, sequence of 0128 (nucleotide numbers 60860-61480 of GeneBank accession number M87049) which is a known but function-unknown ORF. The *rhtC* gene may be obtained by PCR or hybridization using oligonucleotides designed according to the nucleotide sequence of SEQ ID NO: 3. By using olidonucleotides having nucleotide sequence corresponding to a upstream region from nucleotide number 187 and a downstream region from the nucleotide number 804 in SEQ ID NO: 3 as the primers for PCR, the entire coding region can be amplified.

In the present invention, the DNA coding for the RhtB protein of the present invention may code for RhtB protein including deletion, substitution, insertion, or addition of one or several amino acids at one or a plurality of positions, provided that the Rh activity of RhtB protein encoded thereby is not deteriorated. Similarly, the DNA coding for the RhtC protein of the present invention may code for RhtC protein including deletion, substitution, insertion, or addition of one or several amino acids at one or a plurality of positions, provided that the Rt activity of RhtC protein encoded thereby is not deteriorated.

The DNA, which codes for the substantially same protein as the RhtB protein or RhtC protein as described above, may be obtained, for example, by modifying the nucleotide sequence, for example, by means of the sitedirected mutagenesis method so that one or more amino acid residues at a specified site involve deletion, substitution, insertion, or addition. DNA modified as described above may be obtained by the conventionally known mutation treatment. The mutation treatment includes a method for treating a DNA coding for the RhtB protein or RhtC protein *in vitro*, for example, with hydroxylamine, and a method for treating a microorganism, for example, a bacterium, belonging to the genus *Escherichia* harboring a DNA coding for the RhtB protein with ultraviolet irradiation or a mutating agent such as N-methyl-N'-nitro-N-nitrosoquanidine (NTG) and nitrous acid usually used for the mutation treatment.

The DNA, which codes for substantially the same protein as the RhtB protein or RhtC protein, can by obtained by expressing a DNA subjected to *in vitro* mutation treatment as described above in multicopy in an appropriate cell, investigating the resistance to homoserine or threonine, and selecting the DNA which increase the resistance.

It is generally known that an amino acid sequence of a protein and a nucleotide sequence coding for it may be slightly different between species, strains, mutants or variants.

Therefore the DNA, which codes for substantially the same protein as the RhtC protein, can be obtained by isolating a DNA which hybridizes with DNA having, for example, a nucleotide sequence of the nucleotide numbers 187 to 804 of the nucleotide sequence of SEQ ID NO: 3 or a probe obtainable therefrom under stringent conditions, and which codes for a protein having the Rt activity from a bacterium belonging to the genus *Escherihia* which is subjected to mutation treatment, or a spontaneous mutant or a variant of a bacterium belonging to the genus *Escherihia*.

Also, the DNA, which codes for substantially the same protein as the RhtB protein, can be obtained by isolating a DNA which hybridizes with DNA having, for example, a nucleotide sequence of the nucleotide numbers 557 to 1171 of the nucleotide sequence of SEQ ID NO: 1 or a probe obtainable therefrom under stringent conditions, and which codes for a protein having the Rh activity, from a bacterium belonging to the genus *Escherichia* which is subjected to mutation treatment, or a spontaneous mutant or a variant of a bacterium belonging to the genus *Escherichia*.

The term "stringent conditions" referred to herein is a condition under which DNA's are hybridized with each other at a salt concentration corresponding to an ordinary condition of washing in Southern hybridization, i.e., 60 °C, 1 x SSC, 0.1 % SDS, preferably 0.1 x SSC, 0.1 % SDS.

### <2> Bacterium belonging to the genus Escherichia used in the method of the present invention

The bacterium belonging the genus *Escherichia* used in the method of the present invention is a bacterium belonging to the genus *Escherichia* of which the Rt activity is enhanced. Preferred embodiment of the bacterium is a bacterium which is further enhanced the Rh activity. A bacterium belonging to the genus *Escherichia* is exemplified by *Escherichia coli*. The Rt activity can be enhanced by, for example, amplification of the copy number of the *rhtC* structural gene in a cell, or transformation of a bacterium belonging to the genus *Escherihia* with a recombinant DNA in which a DNA fragment including the *rhtC* structural gene encoding the RhtC protein is ligated with a promoter sequence which functions efficiently in a bacterium belonging to the genus *Escherihia*. The Rt activity can be also enhanced by substitution of the promoter sequence of the *rhtC* gene on a chromosome with a promoter sequence which functions efficiently in a bacterium belonging to the genus *Escherichia*.

Besides, the Rh actibity can be enchanced by, for example, amplification of the copy number of the *rhtB* structural gene in a cell, or transformation of a bacterium belonging to the genus *Escherichia* with recombinant DNA in which a DNA fragment including the *rhtB* structural gene encoding RhtB protein is ligated with a promoter sequence which functions efficiently in a bacterium belonging to the genus *Escherichia*. The Rh activity can be also enhanced by substitution of the promoter sequence of the *rhtB* gene on a chromosome with a promoter sequence which functions efficiently in a bacterium belonging to the genus *Escherichia*.

The amplification of the copy number of the *rhtC* structural gene or *rhtB* structural gene in a cell can be performed by introduction of a multicopy vector which carries the *rhtC* structural gene or *rhtB* structural gene into a cell of a bacterium belonging to the genus *Escherihia*. Specifically, the copy number can be increased by introduction of a plasmid, a phage or a transposon (Berg, D.E. and Berg, C.M., Bio/Tecnol., 1, 417 (1983)) which carries the *rhtC* structural gene or *rhtB* structural gene into a cell of a bacterium belonging to the genus *Escherichia*.

The multicopy vector is exemplified by plasmid vectors such as pBR322, pMW118, pUC19 or the like, and phage vectors such as λ1059, λBF101, M13mp9 or the like. The transposon is exemplified by Mu, Tn10, Tn5 or the like.

The introduction of a DNA into a bacterium belonging to the genus *Escherichia* can be performed, for example, by a method of D.A M.Morrison (Methods in Enzymology, 68, 326 (1979)) or a method in which recipient bacterial cell are treated with calcium chloride to increase permeability of DNA (Mandel, M. And Higa, A., J. Mol. Biol., 53, 159, (1970)) and the like.

If the Rt activity, or the Rt activity and the Rh activity is enhanced in an amino acid-producing bacterium belonging to the genus *Escherichia* as described above, a produced amount of the amino acid can be increased. As the bacterium belonging to the genus *Escherichia* which is to be the Rt activity, or the Rt activity and the Rh activity is enhanced, strains which have abilities to produce desired amino acids are used. Besides, an ability to produce an amino acid may be imparted to a bacterium in which the Rt activity, of the Rt activity and Rh activity is enhanced.

On the basis of the rhtC DNA fragment amplification the new strains *E. coli* MG442/pRhtC producing homoserine; *E. coli* MG442/pVIC40,pRhtC producing threonine; *E. coli* NZ10/pRhtBC and *E. coli* NZ10/pRhtB, pRhtC producing homoserine, valine and leucine were obtained which accumulate the amino acids in a higher amount than those containing no amplified rhtC DNA fragment.

The new strains have been deposited (according to international deposition based on Budapest Treaty) in the All-Russian Collection for Industrial Microorganisms (VKPM). The strain *E. coli* MG442/pRhtC has been deposited as an accession number of VKPM B-7700; the strain *E. coli* MG442/pVIC40,pRhtC has been deposited as an accession number of VKPM B-7680; the strain *E. coli* NZ10/pRhtB, pRhtC has been deposited as an accession number of VKPM B-7681, and the strain *E. coli* NZ10/pRhtBC has been deposited as an accession number of VKPM B-7682.

The strain *E. coli* MG442/pRhtC (VKPM B-7700) exhibits the following cultural-morphological and biochemical features.

### Cytomorphology

Gram-negative weakly-motile rods having rounded ends. Longitudinal size, 1.5 to:2 µm.

### Cultural features

### Beef-extract agar:

After 24 hours of growth at 37° C. produces round whitish semitransparent colonies 1.0 to 3 mm in diameter, featuring a smooth surface, regular or slightly wavy edges, the centre is slightly raised, homogeneous structure, pastelike consistency, readily emulsifiable.

### Luria's agar:

After a 24-hour growth at 37° C. develops whitish semitranslucent colonies 1.5 to 2.5 mm in diameter having a smooth surface, homogeneous structure, pastelike consistency, readily emulsifiable.

### Minimal agar-doped medium M9:

After 40 to 48 hours of growth at 37°C forms colonies 0.5 to 1.5 mm in diameter, which are coloured greyish-white, semitransparent, slightly convex, with a lustrous surface.

### Growth in a beaf-extract broth:

After a 24-hour growth at 37° C exhibits strong uniform cloudiness, has a characteristic odour.

### Physiological and biochemical features.

### Grows upon thrust inoculation in a beef-extract agar:

Exhibits good growth throughout the inoculated area. The microorganism proves to be a facultative anaerobe.
It does not liquefy gelatin.
Features a good growth on milk, accompanied by milk coagulation.
Does not produce indole.
Temperature conditions: Grows on beaf-extract broth at 20-42°C, an optimum temperature lying within 33-37 °C.
pH value of culture medium: Grows on liquid media having the pH value from 6 to 8, an optimum value being 7.2.
Carbon sources: Exhibits good growth on glucose, fructose, lactose, mannose, galactose, xylose, glycerol, mannitol to produce an acid and gas.
Nitrogen sources: Assimilates nitrogen in the form of ammonium, nitric acid salts, as well as from some organic compounds.
Resistant to ampicillin.
L-isoleucine is used as a growth factor. However, the strain can grow slowly without isoleucine.
Content of plasmids: The cells contain multicopy hybrid plasmid pRhtC ensuring resistance to ampicillin (100 mg/l) and carrying the rhtC gene responsible for the increased resistance to threonine (50 mg/ml).

The strain *E. coli* MG442/pVIC40, pRhtc (VKPM B-7680) has the same cultural-morphological and biochemical features as the strain VKPM B-7700 except for in addition to pRhtC, it contains a multicopy hybrid plasmid pVIC40 ensuring resistance to streptomycin (100 mg/l) and carrying the genes of the threonine operon.

The strain *E. coli* strain *E. coli* NZ10/pRhtB, pRhtC (VKPM B-7681) has the same cultural-morphological and biochemical features as the strain VKPM B-7700 except for L-threonine (0.1 - 5 mg/ml) is used as a growth factor instead of L-isoleucine. Besides, it contains a multicopy hybride plasmid pRhtB ensuring resistance to kanamycin (50 mg/l) and carrying the rhtB gene which confers resistance to homoserine (10 mg/ml)

The strain *E. coli* strain *E. coli* NZ10/pRhtBC, (VKPM B-7682) has the same cultural-morphological and biochemical features as the strain VKPM B-7681 except for it contains a multicopy hybride plasmid pRhtBC ensuring resistance to ampicillin (100 mg/l) and carrying both the rhtB and rhtC genes which confer resistance to L-homoserine (10 mg/ml) and L-threonine (50mg/ml).

### <3> Method for producing an amino acid

An amino acid can be efficiently produced by cultivating the bacterium in which the Rt activity, or the Rt activity and Rh activity is enhanced by amplifying a copy number of the *rhtC* gene, or *rhtC* gene and *rhtB* gene as described above, and which has an ability to produce the amino acid, in a culture medium, producing and accumulating the amino acid in the medium, and recovering the amino acid from the medium. The amino acid is exemplified preferably by L-homoserine, L-threonine and branched chain amino acids. The branched chain amino acids may be exemplified by L-valine, L-leucine and L-isoleucine, and preferably exemplified by L-valine, L-leucine.

In the method of present invention, the cultivation of the bacterium belonging to the genus *Escherichia*, the collection and purification of amino acids from the liquid medium may be performed in a manner similar to those of the conventional method for producing an amino acid by fermentation using a bacterium. A medium used in cultivation may be either a synthetic medium or a natural medium, so long as the medium includes a carbon and a nitrogen source and minerals and, if necessary, nutrients which the bacterium used requires for growth in appropriate amount. The carbon source may include various carbohydrates such as glucose and sucrose, and various organic acids. Depending on assimilatory ability of the used bacterium. Alcohol including ethanol and glycerol may be used. As the nitrogen source, ammonia, various ammonium salts as ammonium sulfate, other nitrogen compounds such as amines, a natural nitrogen source such as peptone, soybean hydrolyzate and digested fermentative microbe are used. As minerals, monopotassium phosphate, magnesium sulfate, sodium chloride, ferrous sulfate, manganese sulfate, calcium carbonate are used.

The cultivation is preferably culture under an aerobic condition such as a shaking, and an aeration and stirring culture. The temperature of culture is usually 20 to 40°C, preferably 30 to 38°C. The pH of the culture is usually between 5 and 9, preferably between 6.5 and 7.2. the pH of the culture can be adjusted with ammonia, calcium carbonate, various acids, various bases, and buffers. Usually, a 1 to 3-day cultivation leads to the accumulation of the target amino acid in the medium.

Recovering the amino acid can be performed by removing solids such as cells from the medium by centrifugation or membrane filtration after cultivation, and then collecting and purifying the target amino acid by ion exchange, concentration and crystalline fraction methods and the like.

### Brief Explanation of Drawings

Fig. 1 shows cloning and identification of rhtB and rhtC genes,
Fig. 2 shows structure of the plasmid pRhtB harboring *rhtB* gene,
Fig. 3 shows structure of the plasmid pRhtC harboring *rhtC* gene, and
Fig. 4 shows structure of the plasmid pRhtBC harboring *rhtB* gene and *rhtC* gene.

### Best Mode for Carrying Out the Invention

The present invention will be more concretely explained below with reference to Examples. In the Examples, an amino acid is of L-configuration unless otherwise noted.

### Example 1: Obtaining of the rhtB and rhtC DNA fragments

### Step 1.Cloning of genes involving resistance homoserine and threonine into mini-Mu phagemid

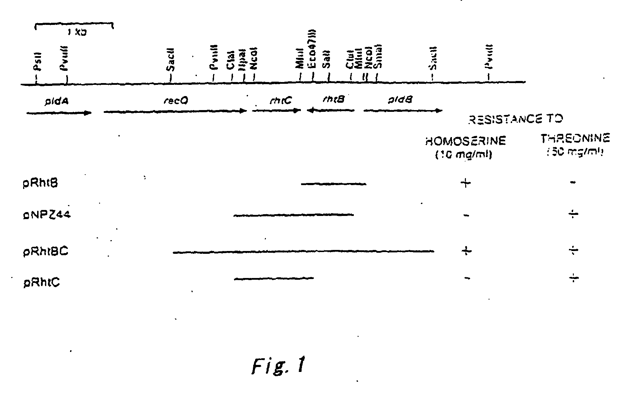

The genes involving resistance homoserine and threonine were cloned *in vivo* using mini-Mu d5005 phagemid (Groisman, E.A., et al., J. Bacteriol., 168, 357-364 (1986)). MuCts62 lysogen of the strain MG442 (Guayatiner et al., Genetika (in Russian), 14, 947-956 (1978))was used as a donor. Freshly prepared lysated were used to infect a Mucts lysogenic derivative of a strain VKPM B-513 (Hfr K10 metB). The cells were plated on M9 glucose minimal medium with methionine (50 µg/ml), kanamycin (40 µg/ml) and homoserine (10 µg/ml). Colonies which appeared after 48 hr were picked and isolated. Plasmid DNA was isolated and used to transform the strain VKPM B-513 by standard techniques. Transformants were selected on L-broth agar plates with kanamycun as above. Plasmid DNA was isolated from those which were resistance to homoserine, and analyzed by restriction mapping of the structure of the inserted fragments. It appeared that two types of inserts belonging to different chromosome regions had been cloned from the donor. Thus, at least two different genes that in multicopy impart resistance to homoserine exist in *E. coli*. One of the two types of inserts is the *rhtA* gene which has already reported (ABSTRACT of 17th International Congress of Biochemistry and Molecular Biology in conjugation with 1997 Annual Meeting af the American Society for Biochemistry and Molecular Biology, San Francisco, California August 24-29, 1997). Among the other of the two types of inserts, a MluI-MluI fragment of 0.8 kb imparts only the resistance to homoserine (Fig. 1).

### Step 2: Identification of rhtB gene and rhtC gene

The insert fragment was sequenced by the dideoxy chain termination method of Sanger. Both DNA strands were sequenced in their entirety and all junctions were overlapped. The sequencing showed that the insert fragment included f138 (nucleotide numbers 61543 to 61959 of GenBank accession number M87049) which was a known but function-unknown ORF (open reading frame) present at 86 min of *E. coli* chlomosome and about 350 bp of an upstream region thereof (downstream region in the sequence of M87049). The f138 which had only 160 nucleotides in the 5'-flanking region could not impart the resistance to homoserine. No termination codon is present upstream the ORF f138 between 62160 and 61950 nucleotides of M87049. Furthermore, one ATG following a sequence predicted as a ribosome binding site is present in the sequence. The larger ORF (nucleotide numbers 62160 to 61546) is designated as *rhtB* gene. The RhtB protein deduced from the gene has a region which is highly hydrophobic and contais possible transmembrane segments.

As described below, the plasmid containing this gene conferred upon cells only the resistance to high concentrations of homoserine. Since the initial *Sac*II-*Sac*II DNA fragment contained the second unidentified ORF, 0128, the gene was subcloned and tested for its ability to confer resistance to homoserine and threonine. It proved that the plasmid containing o128 (*Cla*I-*Eco*47III fragment) conferred resistance to 50 mg/ml threonine (Fig. 1). The subcloned fragment was sequenced and found to contain additional nucleotide (G) in the position between 61213 and 61214 nucleotides of M87049. The nucleotide addition to the sequence eliminated a frame shift and enlarged the ORF into 5'-flanking region up to 60860 nucleotide. This new gene was designated as *rhtC*. Both genes, *rhtB* and *rhtC*, were found to be homologous to transporter involved in lysine export of *Corynebacterium glutamicum*.

### Example 2: The effect of rhtB and rhtC genes amplification on homoserine production.

### <1> Construction of the L-homoserine-producing strain E. coli NZ10/pAL4, pRhtB and homoserine production

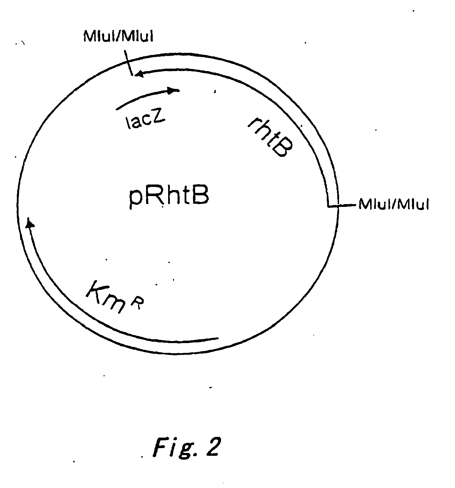

The *rhtB* gene was inserted to a plasmid pUK21 (Vieira, J. And Messing, J., Gene, 100, 189-194 (1991)), to obtain pRhtB (Fig. 2).

Strain NZ10 of *E. coli* was transformed by a plasmid pAL4 which was a pBR322 vector into which the *thrA* gene coding for aspartokinase-homoseine dehydrogenase I was inserted, to obtain the strains NZ10/pAL4. The strain NZ10 is a *leuB⁺*-reverted mutant *thrB⁻* obtained from the *E. coli* strain C600 (*thrB*, *leuB*) (Appleyard R.K., Genetics, 39, 440-452,1954).

The strain NZ10/pAL4 was transformed with pUK21 or pRhtB to obtain strains NZ10/pAL4,pUK21 and NZ10/pAL4, pRhtB.

The thus obtained transformants were each cultivated at 37°C for 18 hours in a nutrient broth with 50 mg/l kanamycin and 100 mg/l ampicilin, and 0.3 ml of the obtained culture was inoculate into 3 ml of a fermentation medium having the following composition and containing 50 mg/l kanamycin and 100 mg/l ampicilin, in a 20 x 200 mm test tube, and cultivated at 37°C for 48 hours with a rotary shaker. After the cultivation, an accumulated amount of homoserine in the medium and an absorbance at 560 nm of the medium were determined by known methods.

**[Fermentation medium composition (g/L)]**

| | |
|---|---|
| Glucose | 80 |
| (NH₄)₂SO₄ | 22 |
| K₂HPO₄ | 2 |
| NaCl | 0.8 |
| MgSO₄•7H₂O | 0.8 |
| FeSO₄•7H₂O | 0.02 |
| MnSO₄•5H₂O | 0.02 |
| Thiamine hydrochloride | 0.2 |
| Yeast Extract | 1.0 |
| CaCO₃ | 30 |
| (CaCO₃ was separately sterilized) | |

The results are shown in Table 1. As shown in Table 1, the strain NZ10/pAL4,pRhtB accumulated homoserine in a larger amount than the strain NZ10/pAL4,pUK21 in which the *rhtB* gene was not enhanced.

**Table 1.**

| Strain | OD₅₆₀ | Accumulated amount of homoserine (g/L) |
|---|---|---|
| NZ10/pAL4, pUK21 | 14.3 | 3.3 |
| NZ10/pAL4,pRhtB | 15.6 | 6.4 |

### <2> Construction of the homoserine-producing strain E. coli MG442/pRhtC and homoserine production

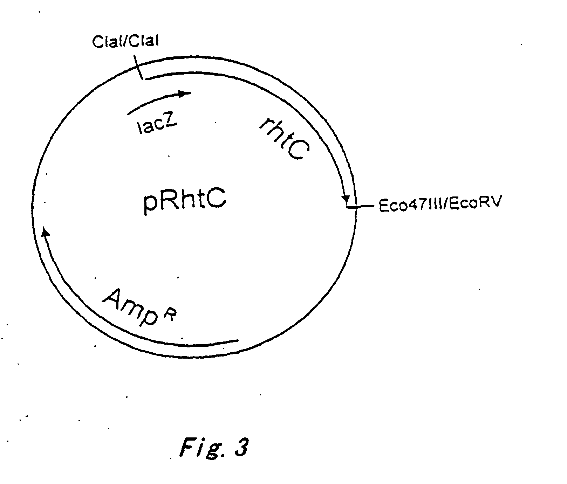

The *rhtC* gene was inserted to pUC21 vector (Vieira, J. And Messing, J., Gene, 100, 189-194 (1991)), to obtain pRhtC (Fig. 3).

The known *E. coli* strain MG442 which can produce threonine in an amount of not less than 3 g/L (Gusyatiner, et al., 1978, Genetika (in Russian), 14:947-956) was transformed by introducing pUC21 or pRhtC to obtain the strains MG442/pUC21 and MG442/pRhtC.

The thus obtained transformants were each cultivated at 37°C for 18 hours in a nutrient broth with 100 mg/ml ampicilin, and 0.3 ml of the obtained culture was inoculate into 3 ml of a fermentation medium describe above and containing 100 mg/ml ampicilin, in a 20 x 200 mm test tube, and cultivated at 37°C for 48 hours with a rotary shaker. After the cultivation, an accumulated amount of homoserine in the medium and an absorbance at 560 nm of the medium were determined by known methods. The results are shown in Table 2.

**Table 2.**

| Strain | OD₅₆₀ | Accumulated amount of homoserine (g/L) |
|---|---|---|
| MG442/pUC21 | 9.7 | <0.1 |
| MG442/pRhtC | 15.2 | 9.5 |

### Example 3: The effect of rhtB and rhtC genes amplification on threonine production.

### <1> Construction of the threonine -producing strain E. coli VG442/pVIC40, pRhtB (VKPM B-7660) and threonine production

The strain MG442 was transformed by introducing a known plasmid pVIC40 (U.S. Patent No. 5,175,107 (1992)) by an ordinary transformation method. Transformants were selected on LB agar plates containing 0.1 mg/ml streptomycin. Thus a novel strain MG442/pVIC40 was obtained.

The strain MG442/pVIC40 was transformed with pUK21 or pRhtB to obtain strain MG442/pVIC40,pUK21 and MG442/pVIC40,pRhtB.

The thus obtained transformants were each cultivated at 37°C for 18 hours in a nutrient broth with 50 mg/l kanamycin and 100 mg/l streptomycin, and 0.3 ml of the obtained culture was inoculate into 3 ml of a fermentation medium describe in Example 2 and containing 50 mg/l kanamycin and 100 mg/l streptomycin, in a 20 x 200 mm test tube, and cultivated at 37°C for 68 hours with a rotary shaker. After the cultivation, an accumulated amount of threonine in the medium and an absorbance at 560 nm of the medium were determined by known methods.

The results are shown in Table 3. As shown in Table 3, the strain MG442/pVIC40,pRhtB accumulated threonine in a larger amount than the strain MG442/pVIC40,pUK21 in which the *rhtB* gene was not enhanced.

**Table 3.**

| Strain | OD₅₆₀ | Accumulated amount of threonine (g/L) |
|---|---|---|
| MG442/pVIC40,pUK21 | 16.3 | 12.9 |
| MG442/pVIC40,pRhtB | 15.2 | 16.3 |

### <2> Construction of the threonine-producing strain E. coli VG442/pVIC40, pRhtC (VKPM B-7680) and threonine production

The strain MG442/pVIC40 was transformed with pRhtC and pUC21. Thus the transformants MG442/pVIC40,pRhtC and MG442/pVIC40, pUC21 were obtained. In the sane manner as describe above, MG442/pVIC40,pUC21 and MG442/pVIC40,pRhtC were each cultivated at 37°C for 18 hours in a nutrient broth with 100 mg/l ampicilin and 100 mg/l streptomycin and 0.3 ml of the obtained culture was inoculate into 3 ml of a fermentation medium describe above and containing 100 mg/l ampicilin and 100 mg/l streptomycin, in a 20 x 200 mm test tube, and cultivated at 37°C for 46 hours with a rotary shaker. After the cultivation, an accumulated amount of threonine in the medium and an absorbance at 560 nm of the medium were determined by known methods.

The results are shown in Table 4. As shown in Table 4, the strain MG442/pVIC40,pRhtC accumulated threonine in a larger amount than the strain MG442/pVIC40,pUC21 in which the *rhtC* gene was not enhanced.

**Table 4**

| Strain | OD₅₆₀ | Accumulated amount of threonine (g/L). |
|---|---|---|
| MG442/pVIC40, pUC21 | 17.4 | 4.9 |
| MG442/pVIC40,pRhtC | 15.1 | 10.2 |

### Example 4: Concerted effect of rhtB gene and rhtC gene on amino acid production

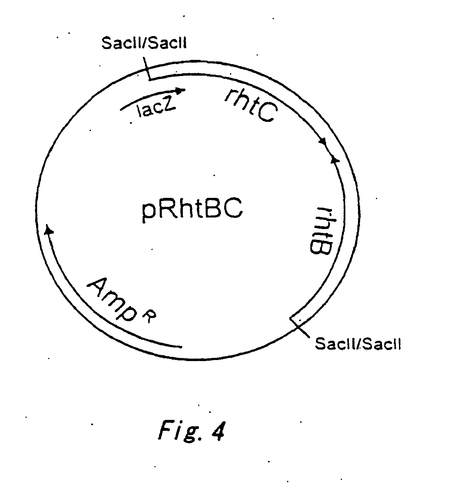

The SacII-SacII DNA fragment containing both *rhtB* and rhtC genes was inserted to the pUC21. Thus the plasmid pRhtBC was obtained which harbors the *rhtB* gene and *rhtC* gene (Fig. 4).

Then, the strain NZ10 was transformed with pUC21, pRhtB, pRhtC or pRhtBC, and the transformants NZ10/pUC21 (VKPM B-7685), NZ10/pRhtB (VKPM B-7683), NZ10/pRhtC (VKPM B-7684), NZ10/pRhtB, pRhtC (VKPM B-7681) and NZ10/pRhtBC (VKPM B-7682) were thus obtained.

The transformants obtained above were cultivated as the same manner as describe above and accumulated amounts of various amino acids in the medium and an absorbance at 540 nm of the medium were determined by known methods.

The result were shown in Table 5. It follows from the Table 5 that concerted effect of the pRhtB and pRhtC on producrion of homoserine, valine and leucine. These results indicate that the *rhtB* and *rhtC* gene products may interact in cells.

**Table 5.**

| Strain | OD₅₆₀ | Homoserine (g/L) | Valine (g/L) | Leucine (g/L) |
|---|---|---|---|---|
| NZ10/pUC21 | 18.7 | 0.6 | 0.22 | 0.16 |
| NZ10/pRhtB | 19.6 | 2.3 | 0.21 | 0.14 |
| NZ10/pRhtC | 20.1 | 0.7 | 0.2 | 0.15 |
| NZ10/pRhtBC | 21.8 | 4.2 | 0.34 | 0.44 |
| NZ10/pRhtB,pRhtC | 19.2 | 4.4 | 0.35 | 0.45 |

### example 5: Effect of rhtB gene and rhtC gene on resistance to amino acids

As describe above, the plasmids harboring the *rhtB* and *rhtC* have positive effect on some amino acid accumulation in culture broth by different strains. It proved that the pattern of accumulated amino acid was dependent on the strain genotype. The homology of the *rhtB* and *rhtC* genes products with the lysine transporter LysE of *Corynebacterium glutamicum* (Vrljic, M., Sahm, H. and Eggeling, L. (1996) Mol. Microbiol. 22, 815-826.) indicates the analogues function for these proteins.

Therefore, the effect of the pRhtB and pRhtC plasmids on susceptibility of the strain N99 which is a streptomycin-resistant (Str^{R}) mutant of the known strain W3350 (VKPM B-1557) to some amino acids and amino acid analogues was tested. Overnight broth cultures (10⁹ cfu/ml) of the strains N99/pUC21, N99pUK21, N99/pRhtB and N99/pRhtC were diluted 1:100 in M9 minimal medium and grown for 5 h in the same medium. Then the log phase cultures thus obtained were diluted and about 10⁴ viable cells were applied to well-dried test plates with M9 agar (2%) containing doubling increments of amino acids or analogues. Thus the minimum inhibitory concentration (MIC) of these compounds were examined.

The result are shown in Table 6. It follows from the Table 6 that multiple copies of *rhtB* besides homoserine conferred increased resistance to α-amino-β-hydroxyvaleric-acid (AHVA) and S-(2-aminoethyl)-L-cysteine (AEC), and 4-aza-DL-leucine; and multiple copies of *rhtC* gene besides threonine increased resistance to valine, histidine, and AHVA. This results indicates that every of the presumed transporters, *RhtB* and *RhtC*, have specificity to several substrates (amino acids), or may shown non-specific effects as a result of amplification.

**Table 6.**

| Substrate | MIC (µg/ml) | | |
|---|---|---|---|
| | N99/pUC21* | N99/pRhtB | N99/pRhtC |
| L-homoserine | 1000 | 20000 | 1000 |
| L-threonine | 30000 | 40000 | 80000 |
| L-valine | 0.5 | 0.5 | 2.0 |
| L-histidine | 5000 | 5000 | 40000 |
| AHVA | 100 | 2000 | 15000 |
| AEC | 5 | 20 | 5 |
| 4-aza-DL-leucine | 50 | 100 | 50 |
| O-methyl-L-threonine | 20 | 20 | 20 |

| | | | |
|---|---|---|---|
| *: The same data were obtain with N99/pUK21. | | | |

## Claims

1. A method for producing the amino acid L-hormoserine or L-threonine, comprising the steps of:
cultivating a bacterium, which has the ability to produce the amino acid, in a culture medium, to produce and
accumulate the amino acid in the medium, and
recovering the amino acid from the medium, said bacterium belonging to the genus *Escherichia,* wherein L-threonine resistance of said bacterium is enhanced by enhancing the activity of a protein as defined in the following (A) or (B) in the cells of said bacterium:
(A) a protein which comprises the amino acid sequence shown in SEQ ID NO: 4 in Sequence Listing; or
(B) a protein which comprises the amino acid sequence including deletion, substitution, insertion or addition of one or several amino acids in the amino acid sequence shown in SEQ ID NO: 4 in Sequence Listing, and which has the activity of making a bacterium having the protein L-threonine-resistant,
wherein the protein is encoded by a DNA which is defined in the following (a) or (b):
(a) a DNA which comprises the nucleotide sequence of nucleotide numbers 187 to 804 in SEO ID NO: 3;
(b) a DNA which hybridizes with a nucleotide sequence of nucleotide numbers 187 to 804 in SEQ ID NO: 3 under a stringent condition and which codes for a protein having the activity of making a bacterium having the protein L-threonine-resistant, wherein the stringent conditions is a condition in which washing is performed at 60 °C and at a salt concentration corresponding to 1 x SSC and 0.1 % SDS.

2. The method according to claim 1, wherein the L-homoserine resistance of said bacterium is further enhanced by enhancing the activity of a protein as defined in the following (C) or (D) in the cells of said bacterium:
(C) a protein which comprises the amino acid sequence shown in SEQ ID NO: 2 in Sequence Listing; or
(D) a protein which comprises the amino acid sequence inducing deletion, substitution, insertion or addition of one or several amino acids in the amino acid sequence shown in SEQ ID NO: 2 in Sequence Listing, and which has the activity of making a bacterium having the protein L-homoserine-resistant.

3. The method according to claim 2, wherein the protein as defined in (C) or (D) is encoded by a DNA which is defined in the following (c) or (d):
(c) a DNA which comprises the nucleotide sequence of nucleotide numbers 557 to 1171 in SEQ ID NO: 1;
(d) a DNA which hybridizes with a nucleotide sequence of nucleotide numbers 557 to 1171 in SEQ ID NO: 1 under a stringent condition and which codes for a protein having the activity of making a bacterium having the protein L-homoserine-resistant wherein the stringent condition is a condition in which washing is performed at 60°C, and at a salt concentration corresponding to 1 x SSC and 0.1 % SDS.

4. A method for producing an amino acid, comprising the steps of:
cultivating a bacterium, which has the ability to produce the amino acid, in a culture medium, to produce and
accumulate the amino add in the medium, and
recovering the amino acid from the medium, said bacterium belonging to the genus *Escherichia,* wherein L-threonine resistance of said bacterium is enhanced by enhancing the activity of a protein as defined in the following (A) or (B) in the cells of said bacterium:
(A) a protein which comprises the amino acid sequence shown in SEQ ID NO: 4 in Sequence Listing; or
(B) a protein which comprises the amino acid sequence including deletion, substitution, insertion or addition of one or several amino acids in the amino add sequence shown in SEQ ID NO: 4 in Sequence Listing, and which has the activity of making a bacterium having the protein L-threonine-resistant,
wherein the protein is encoded by a DNA which is defined in the following (a) or (b):
(a) a DNA which comprises the nucleotide sequence of nucleotide numbers 187 to 804 in SEO ID NO: 3;
(b) a DNA which hybridizes with a nucleotide sequence of nucleotide numbers 187 to 804 in SEQ ID NO: 3 under a stringent condition and which codes for a protein having the activity of making a bacterium having the protein L-threonine-resistant, wherein the stringent conditions is a condition in which washing is performed at 60 °C and at a salt concentration corresponding to 1 x SSC and 0.1 % SDS,
and wherein the L-homoserine resistance of said bacterium is further enhanced by enhancing the activity of a protein as defined in the following (C) or (D) in the cells of said bacterium:
(C) a protein which comprises the amino acid sequence shown in SEQ ID NO: 2 in Sequence Listing; or
(D) a protein which comprises the amino acid sequence including deletion, substitution, insertion or addition of one or several amino acids in the amino acid sequence shown in SEQ ID NO: 2 in Sequence Listing, and which has the activity of making a bacterium having the protein L-homoserine-resistant.

5. The method according to claim 4, wherein said amino acid is branched chain amino acid.

6. The method according to claim 5, wherein said branched chain amino acid is L-valine or L-leucine.

7. The method according to claim 4, wherein the protein as defined in (C) or (D) is encoded by a DNA which is defined in the following (c) or (d):
(c) a DNA which comprises the nucleotide sequence of nucleotide numbers 557 to 1171 in SEQ ID NO: 1;
(d) a DNA which hybridizes with a nucleotide sequence of nucleotide numbers 557 to 1171 in SEQ ID NO: 1 under a stringent condition and which codes for a protein having the activity of making a bacterium having the protein L-homoserine-resistant, wherein the stringent condtion is a condition in which washing is performed at 60°C, and at a salt concentration corresponding to 1 x SSC and 0.1% SDS.

## Patentansprüche

1. Verfahren zum Herstellen der Aminosäure L-Homoserin oder L-Threonin, welches die folgenden Stufen umfasst:
Kultivieren eines Bakteriums, das die Aminosäure produzieren kann, in einem Kulturmedium unter Herstellung und Anhäufung der Aminosäure in dem Medium und
Gewinnen der Aminosäure aus dem Medium,
wobei das Bakterium zur Gattung Escherichia gehört, worin die L-Threonin-Resistenz des Bakteriums durch Verstärken der Aktivität eines Proteins, das nachfolgend in (A) oder (B) definiert ist, in den Zellen des Bakteriums erhöht ist:
(A) ein Bakterium, welches die in SEQ ID Nr. 4 des Sequenzprotokolls gezeigte Aminosäuresequenz aufweist; oder
(B) ein Protein, welches die Aminosäuresequenz, einschließlich Deletion, Substitution, Insertion oder Addition einer oder mehrerer Aminosäuren in der in SEQ ID Nr. 4 des Sequenzprotokolls gezeigten Aminosäuresequenz aufweist und die Wirkung hat, ein das Protein enthaltende Bakterium L-Threonin-resistent zu machen, wobei das Protein von einer DNA, die nachfolgend in (A) oder (B) definiert ist, kodiert wird:
(a) eine DNA, welche die Nucleotidsequenz der Nucleotidnummern 187 bis 804 in der SEQ ID Nr. 3 enthält;
(b) eine DNA, welche mit einer Nucleotidsequenz der Nucleotidnummern 187 bis 804 in der SEQ ID Nr. 3 unter einer stringenten Bedingung hybridisiert und für ein Protein mit der Wirkung kodiert, ein das Protein enthaltende Bakterium L-Threonin-resistent zu machen, wobei die stringente Bedingung eine Bedingung ist, bei der das Waschen bei 60°C bei einer Salzkonzentration, die 1 x SSC und 0,1 % SDS entspricht, durchgeführt wird.

2. Verfahren nach Anspruch 1, wobei die L-Homoserin-Resistenz des Bakteriums außerdem durch Verstärken der Aktivität eines nachfolgend in (C) oder (D) definierten Proteins in den Zellen des Bakteriums erhöht ist:
(C) ein Protein, welches die in SEQ ID Nr. 2 des Sequenzprotokolls gezeigte Aminosäuresequenz aufweist; oder
(D) ein Protein, welches die Aminosäuresequenz, einschließlich Deletion, Substitution, Insertion oder Addition einer oder mehrerer Aminosäuren in der in SEQ ID Nr. 2 des Sequenzprotokolls gezeigten Aminosäuresequenz aufweist und die Wirkung hat, ein das Protein enthaltende Bakterium L-Homoserin-resistent zu machen.

3. Verfahren nach Anspruch 2, wobei das in (C) oder (D) definierte Protein von einer DNA kodiert wird, die nachfolgend in (c) oder (d) definiert ist:
(c) eine DNA, welche die Nucleotidsequenz der Nucleotidnummern 557 bis 1171 in der SEQ ID Nr. 1 enthält;
(d) eine DNA, welche mit einer Nucleotidsequenz der Nucleotidnummern 557 1171 in SEQ ID Nr. 1 unter einer stringenten Bedingung hybridisiert und für ein Protein mit der Wirkung kodiert, ein das Protein enthaltende Bakterium L-Homoserin-resistent zu machen, wobei die stringente Bedingung eine Bedingung ist, bei der das Waschen bei 60°C und einer Salzkonzentration, die 1 x SSC und 0,1% SDS entspricht, durchgeführt wird.

4. Verfahren zum Herstellen einer Aminosäure, welches die folgenden Stufen umfasst:
Kultivieren eines Bakteriums, das die Aminosäure produzieren kann, in einem Kulturmedium unter Herstellung und Anhäufung der Aminosäure in dem Medium und
Gewinnen der Aminosäure aus dem Medium,
wobei das Bakterium zur Gattung Escherichia gehört, worin die L-Threonin-Resistenz des Bakteriums durch Verstärken der Aktivität eines Proteins, das nachfolgend in (A) oder (B) definiert ist, in den Zellen des Bakteriums erhöht ist:
(A) ein Bakterium, welches die in SEQ ID Nr. 4 des Sequenzprotokolls gezeigte Aminosäuresequenz aufweist; oder
(B) ein Protein, welches die Aminosäuresequenz, einschließlich Deletion, Substitution, Insertion oder Addition einer oder mehrerer Aminosäuren in der in SEQ ID Nr. 4 des Sequenzprotokolls gezeigten Aminosäuresequenz aufweist und die Wirkung hat, ein das Protein enthaltende Bakterium L-Threonin-resistent zu machen, wobei das Protein von einer DNA, die nachfolgend in (A) oder (B) definiert ist, kodiert wird:
(a) eine DNA, welche die Nucleotidsequenz der Nucleotidnummern 187 bis 804 in der SEQ ID Nr. 3 enthält;
(b) eine DNA, welche mit einer Nucleotidsequenz der Nucleotidnummern 187 bis 804 in der SEQ ID Nr. 3 unter einer stringenten Bedingung hybridisiert und für ein Protein mit der Wirkung kodiert, ein das Protein enthaltende Bakterium L-Threonin-resistent zu machen, wobei die stringente Bedingung eine Bedingung ist, bei der das Waschen bei 60°C bei einer Salzkonzentration, die 1 x SSC und 0,1 % SDS entspricht, durchgeführt wird und wobei die L-Homoserin-Resistenz des Bakteriums außerdem durch Verstärken der Aktivität eines nachfolgend in (C) oder (D) definierten Proteins in den Zellen des Bakteriums erhöht ist:
(C) ein Protein, welches die in SEQ ID Nr. 2 des Sequenzprotokolls gezeigte Aminosäuresequenz aufweist; oder
(D) ein Protein, welches die Aminosäuresequenz, einschließlich Deletion, Substitution, Insertion oder Addition einer oder mehrerer Aminosäuren in der in SEQ ID Nr. 2 des Sequenzprotokolls gezeigten Aminosäuresequenz aufweist und die Wirkung hat, ein das Protein enthaltende Bakterium L-Homoserin-resistent zu machen.

5. Verfahren nach Anspruch 4, wobei die Aminosäure eine verzweigte Aminosäure ist.

6. Verfahren nach Anspruch 5, wobei die verzweigte Aminosäure L-Valin oder L-Leucin ist.

7. Verfahren nach Anspruch 4, wobei das in (C) oder (D) definierte Protein von einer DNA kodiert wird, die nachfolgend in (c) oder (d) definiert ist:
(c) eine DNA, welche die Nucleotidsequenz der Nucleotidnummern 557 bis 1171 in der SEQ ID Nr. 1 enthält;
(d) eine DNA, welche mit einer Nucleotidsequenz der Nucleotidnummern 557 1171 in SEQ ID Nr. 1 unter einer stringenten Bedingung hybridisiert und für ein Protein mit der Wirkung kodiert, ein das Protein enthaltende Bakterium L-Homoserin-resistent zu machen, wobei die stringente Bedingung eine Bedingung ist, bei der das Waschen bei 60°C und einer Salzkonzentration, die 1 x SSC und 0,1% SDS entspricht, durchgeführt wird.

## Revendications

1. Procédé pour produire l'aminoacide L-homosérine ou L-thréonine comprenant les étapes de :
cultiver une bactérie qui a l'aptitude à produire l'aminoacide, dans un milieu de culture, pour produire et accumuler l'aminoacide dans le milieu, et
récupérer l'aminoacide à partir du milieu, ladite bactérie appartenant au genre *Escherichia,* où la résistance à la L-thréonine de ladite bactérie est augmentée par augmentation de l'activité d'une protéine telle que définie en (A) ou (B) suivant dans les cellules de ladite bactérie :
(A) une protéine qui comprend la séquence d'aminoacides montrée dans SEQ ID NO : 4 dans le listage de séquences ; ou
(B) une protéine qui comprend la séquence d'aminoacides incluant une délétion, une substitution, une insertion ou une addition d'un ou plusieurs aminoacides dans la séquence d'aminoacides montrée dans SEQ ID NO : 4 dans le listage de séquences, et qui a l'activité de rendre une bactérie ayant la protéine résistante à la L-thréonine,
où la protéine est codée par un ADN qui est défini en (a) ou (b) suivant :
(a) un ADN qui comprend la séquence nucléotidique de numéros de nucléotides 187 à 804 dans SEQ ID NO : 3 ;
(b) un ADN qui s'hybride avec une séquence nucléotidique de numéros de nucléotides 187 à 804 dans SEQ ID NO : 3 dans des conditions stringentes et qui code une protéine ayant l'activité de rendre une bactérie ayant la protéine résistante à la L-thréonine, où les conditions stringentes sont des conditions dans lesquelles un lavage est réalisé à 60°C, et à une concentration de sels correspondant à 1 x SSC et 0,1 % de SDS.

2. Procédé selon la revendication 1 où la résistance à la L-homosérine de ladite bactérie est augmentée encore par augmentation de l'activité d'une protéine telle que définie en (C) ou (D) suivant dans les cellules de ladite bactérie :
(C) une protéine qui comprend la séquence d'aminoacides montrée dans SEQ ID NO : 2 dans le listage de séquences ; ou
(D) une protéine qui comprend la séquence d'aminoacides incluant une délétion, une substitution, une insertion ou une addition d'un ou plusieurs aminoacides dans la séquences d'aminoacides montrée dans SEQ ID NO : 2 dans le listage de séquences, et qui a l'activité de rendre une bactérie ayant la protéine résistante à la L-homosérine.

3. Procédé selon la revendication 2 où la protéine telle que définie en (C) ou (D) est codée par un ADN qui est défini en (c) ou (d) suivant :
(c) un ADN qui comprend la séquence nucléotidique de numéros de nucléotides 557 à 1171 dans SEQ ID NO : 1 ;
(d) un ADN qui s'hybride avec une séquence nucléotidique de numéros de nucléotides 557 à 1171 dans SEQ ID NO: 1 dans des conditions stringentes et qui code une protéine ayant l'activité de rendre une bactérie ayant la protéine résistante à la L-homosérine, où les conditions stringentes sont des conditions dans lesquelles un lavage est réalisé à 60°C, et à une concentration de sels correspondant à 1 x SSC et 0,1 % de SDS.

4. Procédé pour produire un aminoacide comprenant les étapes de :
cultiver une bactérie qui a l'aptitude à produire l'aminoacide, dans un milieu de culture, pour produire et accumuler l'aminoacide dans le milieu, et
récupérer l'aminoacide à partir du milieu, ladite bactérie appartenant au genre *Escherichia,* où la résistance à la L-thréonine de ladite bactérie est augmentée par augmentation de l'activité d'une protéine telle que définie en (A) ou (B) suivant dans les cellules de ladite bactérie :
(A) une protéine qui comprend la séquence d'aminoacides montrée dans SEQ ID NO : 4 dans le listage de séquences ; ou
(B) une protéine qui comprend la séquence d'aminoacides incluant une délétion, une substitution, une insertion ou une addition d'un ou plusieurs aminoacides dans la séquence d'aminoacides montrée dans SEQ ID NO : 4 dans le listage de séquences, et qui a l'activité de rendre une bactérie ayant la protéine résistante à la L-thréonine,
où la protéine est codée par un ADN qui est défini en (a) ou (b) suivant :
(a) un ADN qui comprend la séquence nucléotidique de numéros de nucléotides 187 à 804 dans SEQ ID NO : 3 ;
(b) un ADN qui s'hybride avec une séquence nucléotidique de numéros de nucléotides 187 à 804 dans SEQ ID NO : 3 dans des conditions stringentes et qui code une protéine ayant l'activité de rendre une bactérie ayant la protéine résistante à la L-thréonine, où les conditions stringentes sont des conditions dans lesquelles un lavage est réalisé à 60°C, et à une concentration de sels correspondant à 1 x SSC et 0,1 % de SDS, et
où la résistance à la L-homosérine de ladite bactérie est augmentée encore par augmentation de l'activité d'une protéine telle que définie en (C) ou (D) suivant dans les cellules de ladite bactérie :
(C) une protéine qui comprend la séquence d'aminoacides montrée dans SEQ ID NO : 2 dans le listage de séquences ; ou
(D) une protéine qui comprend la séquence d'aminoacides incluant une délétion, une substitution, une insertion ou une addition d'un ou plusieurs aminoacides dans la séquences d'aminoacides montrée dans SEQ ID NO : 2 dans le listage de séquences, et qui a l'activité de rendre une bactérie ayant la protéine résistante à la L-homosérine.

5. Procédé selon la revendication 4 où ledit aminoacide est un aminoacide ramifié.

6. Procédé selon la revendication 5 où ledit aminoacide ramifié est la L-valine ou la L-leucine.

7. Procédé selon la revendication 4 où la protéine telle que définie en (C) ou (D) est codée par un ADN qui est défini en (c) ou (d) suivant :
(c) un ADN qui comprend la séquence nucléotidique de numéros de nucléotides 557 à 1171 dans SEQ ID NO : 1 ;
(d) un ADN qui s'hybride avec une séquence nucléotidique de numéros de nucléotides 557 à 1171 dans SEQ ID NO: 1 dans des conditions stringentes et qui code une protéine ayant l'activité de rendre une bactérie ayant la protéine résistante à la L-homosérine, où les conditions stringentes sont des conditions dans lesquelles un lavage est réalisé à 60°C, et à une concentration de sels correspondant à 1 x SSC et 0,1 % de SDS.
